# EUROPÄISCHE PATENTSCHRIFT

(11) **EP 2 699 311 B1**
(45) Veröffentlichungstag und Bekanntmachung des Hinweises auf die Patenterteilung: **17.12.2014**
(21) Anmeldenummer: 12720408.9
(22) Anmeldetag: 16.04.2012
(51) Int. Cl.: A61N 1/36, A61N 1/04, A61H 39/00, A61H 39/02

(54) **VORRICHTUNG ZUR AUFBRINGUNG EINES TRANSKUTANEN ELEKTRISCHEN STIMULATIONSREIZES AUF DIE OBERFLÄCHE EINES ABSCHNITTS DES MENSCHLICHEN OHRS**
DEVICE FOR APPLYING A TRANSCUTANEOUS ELECTRICAL STIMULATION TO THE SURFACE OF A SECTION OF THE HUMAN EAR
DISPOSITIF DESTINÉ À SOUMETTRE LA SURFACE D'UNE PARTIE DE L'OREILLE HUMAINE À UN SIGNAL DE STIMULATION ÉLECTRIQUE TRANSCUTANÉE

(30) Priorität: 19.04.2011 DE 102011018228
(43) Veröffentlichungstag der Anmeldung: 26.02.2014
(73) Patentinhaber: Cerbomed GmbH, 91052 Erlangen (DE)
(72) Erfinder: ELLRICH, Jens, 91094 Langensendelbach (DE); BECK, Christoph, 91096 Möhrendorf (DE); FRENKEL, Wolf Gerhard, 72514 Inzigkofen-Engelswies (DE); HARTLEP, Andreas, 83607 Holzkirchen (DE)
(74) Vertreter: Gosdin, Michael
(86) Internationale Anmeldenummer: PCT/EP2012/001633
(87) Internationale Veröffentlichungsnummer: WO 2012/143111

(56) Entgegenhaltungen:
- WO-A1-92/08516
- WO-A2-2008/128270
- WO-A2-2009/137683
- WO-A2-2010/032114
- DE-A1-102010 015 278
- DE-B3-102010 054 165
- US-A- 3 659 614
- US-A1- 2003 195 588
- US-B1- 6 341 237

## Beschreibung

Die Erfindung betrifft eine Vorrichtung zur Aufbringung eines transkutanen elektrischen Stimulationsreizes auf die Oberfläche eines Abschnittes des menschlichen Ohrs, die ein am oder im Ohr anbringbares Halteelement sowie eine Anzahl Elektroden aufweist, die an oder in einem Elektrodenträger angeordnet sind, wobei die Vorrichtung eine Steuerungseinrichtung umfasst, die die Herstellung einer Potentialdifferenz zwischen den Elektroden steuert oder regelt, wobei mindestens drei Elektroden am oder im Elektrodenträger angeordnet sind, wobei sich die mindestens drei Elektroden in einer Ebene befinden. Des weiteren betrifft die Erfindung ein Verfahren zum Betrieb einer solchen Vorrichtung.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-in vasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Vorrichtung der eingangs genannten Art ist aus der WO 2010/032114 A2 bekannt. Hier wird ein Kopfhörer beschrieben, der in den Gehörkanal eingesetzt werden kann, wobei am Kopfhörer Elektroden angeordnet sind, um eine transkutane Elektrostimulation vornehmen zu können. Andere Lösungen werden in der US 3 659 614, in der US 6 341 237 B1 und in der WO 2009/137683 A2 offenbart**.**

Eine andere Vorrichtung ist aus der DE 10 2006 023 824 B4 bekannt. Hier ist eine Vorrichtung zur transkutanen Stimulation des Vagusnervs des menschlichen Körpers beschrieben, die in diesem konkreten Falle in der Pinna des Ohres angeordnet werden kann. Die transkutane Stimulation des Vagusnervs erfolgt, nachdem Parameter des Stimulationsstroms vorgegeben wurden. Die Daten können den individuellen Bedürfnissen zwar angepasst werden. Nach der Einstellung der Daten liegen diese indes fest. Die Kontaktierung des zu stimulierenden Gewebes erfolgt mittels zweier kugelförmiger Elektroden, die elastisch gegen die Hautoberfläche verspannt werden.

Es hat sich gezeigt, dass die Applikation transkutaner Stimulationsreize insbesondere im Bereich der Cymba conchae vorteilhaft ist. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Im Unterschied zu der genannten DE 10 2006 023 824 B4 ist es hier allerdings schwieriger, einen einwandfreien elektrischen Kontakt zwischen Stimulationselektroden und Hautoberfläche herzustellen. Hier kann in besonderer Weise eine fettige Hautoberfläche oder eine Behaarung vorliegen, die die Herstellung eines guten elektrischen Kontakts erschwert.

Der vorliegenden Erfindung liegt daher die **Aufgabe** zugrunde, eine Vorrichtung der genannten Art sowie ein zugehöriges Verfahren bereitzustellen, die es bzw. das es in verbesserter Weise erlaubt, eine sichere transkutane Stimulation zu bewerkstelligen. Dabei soll auch gegebenenfalls in einfacher Weise festgestellt werden können, dass die Kontaktverhältnisse der Elektroden auf der Haut ungenügend sind.

Die **Lösung** dieser Aufgabe durch die Erfindung ist im Anspruch 1 definiert und ist dadurch gekennzeichnet, dass mindestens eine der mindestens drei Elektroden in ihrer Position am Elektrodenträger einstellbar angeordnet ist, wobei die mindestens eine in ihrer Position einstellbare Elektrode in der Ebene translatorisch verschieblich angeordnet ist, wobei der Elektrodenträger relativ zum Halteelement beweglich angeordnet ist, so dass der Elektrodenträger bei am Ohr angebrachtem Halteelement relativ zum Halteelement so verschwenken kann, dass sich alle Elektroden im Kontakt mit der Hautoberfläche der Cymba conchae befinden, wobei zwischen dem Elektrodenträger und dem Halteelement ein Kugelgelenk oder ein Scharniergelenk angeordnet ist oder wobei zwischen dem Elektrodenträger und dem Halteelement ein Federelement (vorzugsweise als Verbindungsabschnitt aus dauerelastischem Material ausgebildet) angeordnet ist.

Die abhänhigen Ansprüche betreffen bevorzugte Ausführungen der Erfindung.

Bevorzugt sind genau drei Elektroden am Elektrodenträger angeordnet.

Die genannte Ebene entspricht dabei insbesondere zumindest näherungsweise der Hautoberfläche, auf die ein transkutaner Stimulationsreiz ausgeübt werden soll.

Dabei ist bevorzugt vorgesehen, dass drei Elektroden an oder in dem Elektrodenträger dreieckförmig angeordnet sind, insbesondere in Form eines gleichseitigen Dreiecks.

Vorzugsweise sind alle Elektroden in ihrer Position am Elektrodenträger einstellbar angeordnet. Die mindestens eine in ihrer Position einstellbare Elektrode ist bevorzugt in einer Linearführung linearverschieblich angeordnet. Dabei sind vorzugsweise bei Verstellbarkeit aller Elektroden die Linearführungen zueinander sternförmig ausgerichtet, d. h. die Elektroden können radial verschoben werden. Zumindest eine der Elektroden kann auch in einer Führung verschiebbar angeordnet sein, die bogenförmig um einen gedachten Zentrumspunkt verläuft; damit kann diese Elektrode auf einer Kreisbahn verschoben und so den Nachbarelektroden angenähert bzw. von ihnen entfernt werden. Damit wird die Lage der Elektroden für jede individuelle Anwendung optimal anpassbar.

Bevorzugt ist die Verschwenkbarkeit des Elektrodenträgers relativ zum Halteelement um eine Längsachse des Halteelements unterbunden.

Die Elektroden sind bevorzugt als halbkugelförmige Metallelemente ausgebildet; aber auch andere Gestaltungen sind natürlich möglich.

Das Verfahren zum Betrieb einer solchen Vorrichtung zur Aufbringung eines transkutanen elektrischen Stimulationsreizes auf die Oberfläche eines Abschnittes des menschlichen Ohrs zeichnet sich durch die Verfahrensschritte aus:
a) Messen des Widerstandes zwischen jeweils einem Paar der Elektroden bei am oder im Ohr angebrachter Vorrichtung für alle möglichen Paare von Elektroden durch die Steuerungseinrichtung;
b) Vergleich der gemessenen Widerstände zwischen den Paaren der Elektroden und Auswählen des geringsten Widerstands durch die Steuerungseinrichtung;
c) Veranlassen einer vorgegebenen transkutanen Stimulation durch die Steuerungseinrichtung über das Paar an Elektroden, die zwischen sich den geringsten Widerstand aufweisen.

Die Steuerungseinrichtung kann dabei nach der Durchführung des Schritts a) die gemessenen Widerstände mit einem gespeicherten Referenzwiderstand vergleichen, wobei ein Signal ausgegeben wird und/oder die Stimulation unterbunden wird, falls alle gemessenen Widerstände über dem gespeicherten Referenzwiderstand liegen.

Die erfindungsgemäß vorgeschlagene Otoplastik (Stimulationsvorrichtung) hat also mindestens - vorzugsweise genau - drei punktförmige Elektroden, die in einer Ebene in der Cymba conchae angeordnet sind.

Von der Steuerungseinrichtung aus werden die Elektroden mit einem Stimulationsstrom beaufschlagt.

Die drei punktförmigen Elektroden können wie die Eckpunkte eines Dreiecks angeordnet sein. Hierdurch ergibt sich auch bei einem geringen Andruck des Elektrodenträgers auf die Hautoberfläche eine gute Auflage aller drei Elektroden an der Haut.

Der Elektrodenträger kann, wie erläutert, mit einer flexiblen Einstellung auf die Unebenheiten des Untergrundes ausgestattet sein (analog zu einem Eisenbahn-Drehgestell mit mittigem Drehpunkt, wodurch eine Einstellung auf Unebenheiten möglich wird). Hierfür kommt eine Lösung mit einem Scharnier oder einem Kugelgelenk in Frage, wobei bevorzugt eine Einschränkung der Bewegungsfreiheitsgrade dahingehend vorgesehen ist, dass keine axiale Verdrehung um die Längsachse des Halteelements möglich ist.

Möglich ist auch ein dauerelastischer Puffer zwischen Elektrodenträger und Halteelement (Andruckbügel) mit einer gewissen Rückstellkraft.

Das oben genannte Verfahren erlaubt die Messung der Kontaktqualität mit einer Kontaktbewertung an allen (drei) Elektroden; im Anschluss erfolgt ein Ausschluss der am schlechtesten leitenden Elektrode und eine anschließende Stimulation nur mit den beiden anderen, besser übertragenden Elektroden.

Die genannte Kontaktbewertung kann anhand eines fest abgespeicherten Referenzwertes erfolgen, der in der Steuerungseinrichtung hinterlegt ist.

Eine mögliche Betriebsweise stellt auf die gleichzeitige Beaufschlagung der insbesondere drei Elektroden mit im oder gegen den Uhrzeigersinn sich drehenden Polungsänderungen oder Stimulationsabfolgen ab.

Vorteilhaft ist auch eine variable Anbringung zumindest einer der mindestens drei Elektroden hinsichtlich des Abstands relativ zu den (beiden) anderen Elektroden. Vorzugsweise kann eine solche Einstellbarkeit für alle Elektroden vorgesehen werden. Das Ziel ist dabei die Abdeckung eines optimalen Stimulationsbereiches für jedes individuelle Ohr.

In der Zeichnung ist ein Ausführungsbeispiel der Erfindung dargestellt. Es zeigen:
- Fig. 1: schematisch in perspektivischer Ansicht eine Stimulationsvorrichtung zur transkutanen Stimulation der Cymba conchae eines menschlichen Ohrs,
- Fig. 2: schematisch in perspektivischer Ansicht die Stimulationsvorrichtung nach Fig. 1, gesehen aus einem anderen Blickwinkel, und
- Fig. 3: ein menschliches Ohr, an dem die Stimulationsvorrichtung angeordnet ist.

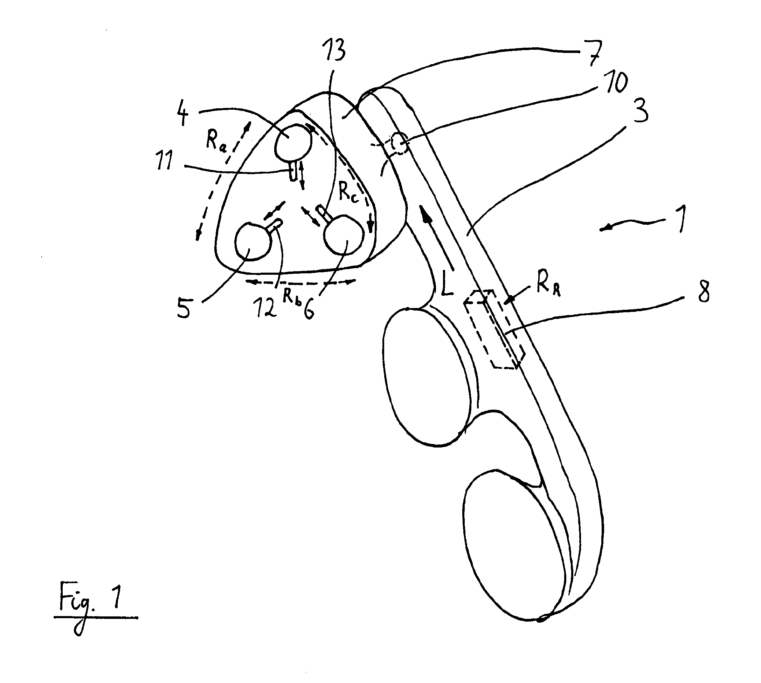

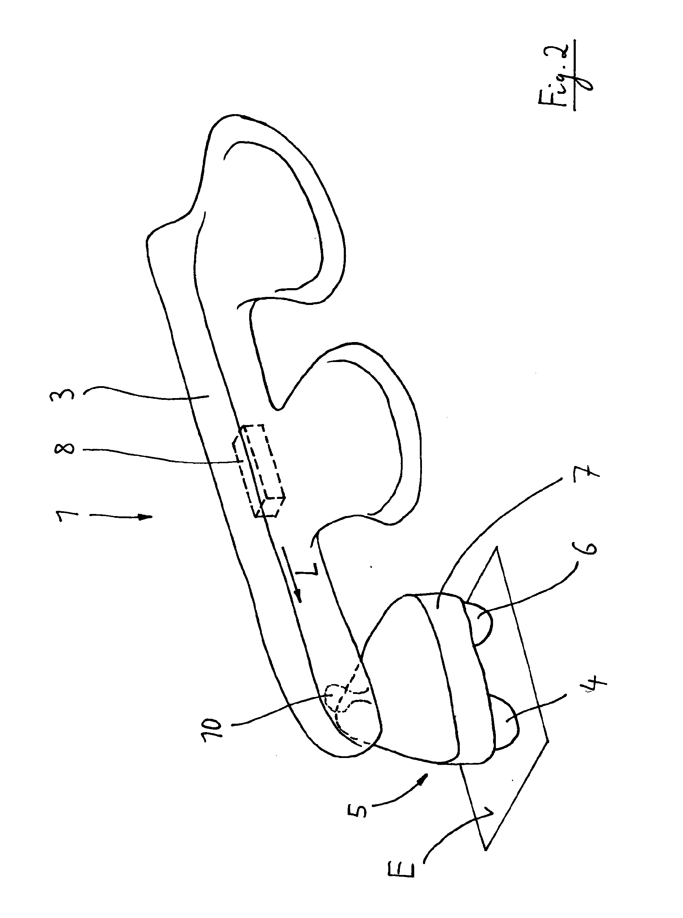

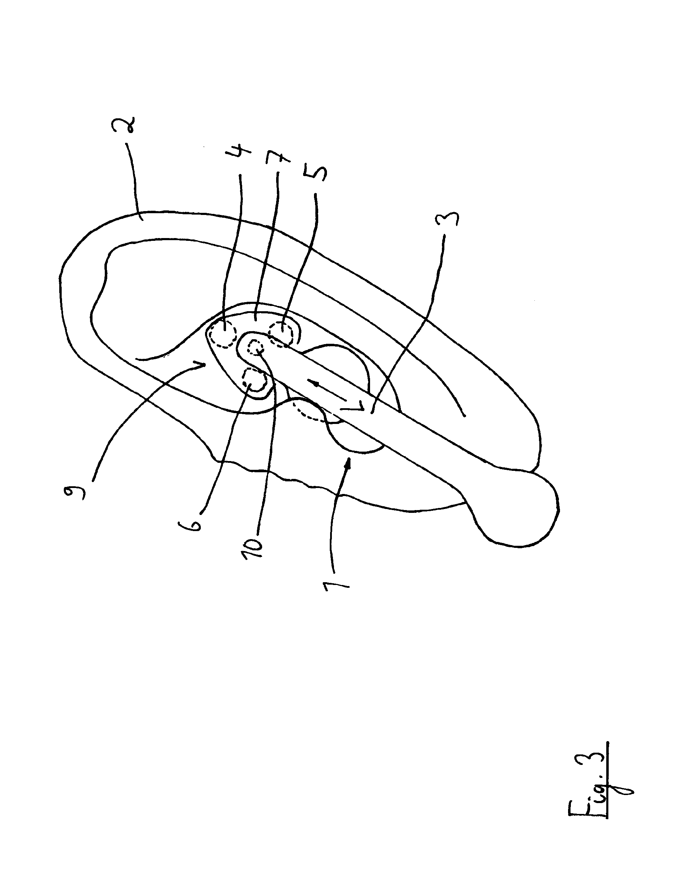

In den Figuren 1 und 2 ist eine Vorrichtung 1 zur transkutanen Stimulation eines Abschnitts des menschlichen Ohrs skizziert. Die Vorrichtung 1 weist ein Halteelement 3 auf, das am Ohr festgelegt werden kann (s. hierzu auch Fig. 3). Das Halteelement 3 erstreckt sich im wesentlichen in Richtung einer Längsachse L. Am Halteelement 3 ist ein Elektrodenträger 7 angeordnet, der vorliegend drei Elektroden 4, 5 und 6 aufweist. Die Elektroden sind vorliegend als halbkugelförmige Strukturen ausgebildet.

Was den prinzipiellen Aufbau einer Vagusnerv-Stimulationsvorrichtung anbelangt, entspricht der beschriebene Aufbau der vorbekannten Lösung gemäß der oben genannten DE 10 2006 023 824 B4 der Anmelderin, auf die insoweit ausdrücklich Bezug genommen wird.

Die Elektrodenanordnung ist ausgebildet, um im Bereich des Vagusnervs am Ohr der die Vorrichtung 1 benutzenden Person angebracht zu werden. Damit kann eine transkutane Stimulation des Vagusnervs vorgenommen werden.

Wie sich aus der Zusammenschau von Fig. 1 und Fig. 2 ergibt, liegen die drei Elektroden 4, 5, 6 in einer Ebene E. Sie sind dreieckförmig angeordnet. Dies hat den Vorteil, dass eine statisch bestimmte Auflage auf der zu stimulierenden Oberfläche gegeben ist, wodurch sich alle drei Elektroden 4, 5, 6 optimal auf die Hautoberfläche legen.

Die Versorgung der Elektroden 4, 5, 6 mit einem Stimulationsstrom wird über eine Steuerungseinrichtung 8 veranlasst, die nur sehr schematisch angedeutet ist.

Wie weiter unter Einbeziehung von Fig. 3 zu erkennen ist, Wird die Vorrichtung 1 so im bzw. am Ohr 2 eines Patienten platziert, dass der Elektrodenträger 7 mit seinen drei Elektroden 4, 5, 6 im Bereich der Cymba conchae zu liegen kommt. Der Elektrodenträger 7 ist relativ zum Halteelement 3 mittels eines Kugelgelenks 10 derart verschwenkbar angeordnet, dass er sich der Hautoberfläche 9 des Ohrs 2 bzw. der Cymba conchae anpassen kann. Demgemäß kann sich der Elektrodenträger 7 in seiner Position so anpassen, dass alle drei Elektroden 4, 5, 6 optimal auf der Hautoberfläche 9 aufliegen.

Eine optimale Anpassung der Lage der Elektroden 4, 5, 6 an die Größe der Cymba conchae wird dadurch ermöglicht, dass die Elektroden 4, 5, 6 mittels jeweiliger Linearführungen 11, 12, 13 in Richtung des Doppelpfeils einstellbar angeordnet sind (s. Fig. 1). Die Linearführungen 11, 12, 13 müssen dabei nicht zwingend eine absolut geradlinige Verschiebung der Elektroden 4, 5, 6 erlauben; möglich ist auch eine leicht bogenförmige, aber immer noch im wesentlichen lineare Verschiebung der Elektroden.

Die Steuerungseinrichtung 8, die über nicht dargestellte Drähte mit den Elektroden 4, 5 und 6 in Verbindung steht, ist ausgebildet, dasjenige Paar von Elektroden zu ermitteln, zwischen denen der geringste elektrische Widerstand vorliegt. Über dieses Paar erfolgt dann die Stimulation.

Hierfür misst die Steuerungseinrichtung 8 vor der eigentlichen Stimulation den elektrischen Widerstand zwischen den vorliegend drei Elektrodenpaaren, d. h. zwischen den Elektroden 4 und 5 (Widerstand Rₐ), zwischen den Elektroden 5 und 6 (Widerstand R_{b}) und zwischen den Elektroden 4 und 6 (Widerstand R_{c}) - s. hierzu Fig. 1. In der Steuerungseinrichtung 8 selber ist ein Referenzwiderstand R_{R} gespeichert.

Eine ordnungsgemäße Elektrostimulation ist möglich, wenn jedenfalls einer der Widerstände Rₐ, R_{b} bzw. R_{c} unterhalb des vorgegebenen Referenzwiderstands R_{R} liegt.

Die Steuerungseinrichtung 8 ist ausgebildet, nach der Messung der Widerstände Rₐ, R_{b} und R_{c} die Messwerte mit dem Referenzwiderstand R_{R} zu vergleichen.

Sind alle drei gemessenen Widerstände höher als der Referenzwiderstand, liegen ungünstige Kontaktverhältnisse vor, so dass in diesem Falle von der Steuerungseinrichtung 8 die Ausgabe eines (Warn)Signals veranlasst werden kann und/oder die Steuerungseinrichtung 8 unterbindet (vorsorglich) die Erzeugung eines Stimulationsstroms. In diesem Falle müssen die Elektroden neu appliziert werden, beispielsweise nach Reinigung des Bereichs der Cymba conchae, der elektrostimuliert werden soll.

### Bezugszeichenliste:

- 1: Vorrichtung zur transkutanen Stimulation
- 2: Ohr
- 3: Halteelement
- 4: Elektrode
- 5: Elektrode
- 6: Elektrode
- 7: Elektrodenträger
- 8: Steuerungseinrichtung
- 9: Hautoberfläche
- 10: Kugelgelenk
- 11: Linearführung
- 12: Linearführung
- 13: Linearführung

- E: Ebene
- L: Längsachse
- Rₐ: Widerstand
- R_{b}: Widerstand
- R_{c}: Widerstand
- R_{R}: Referenzwiderstand

## Patentansprüche

1. Vorrichtung (1) zur Aufbringung eines transkutanen elektrischen Stimulationsreizes auf die Oberfläche eines Abschnittes des menschlichen Ohrs (2), die ein am oder im Ohr (2) anbringbares Halteelement (3) sowie eine Anzahl Elektroden (4, 5, 6) aufweist, die an oder in einem Elektrodenträger (7) angeordnet sind, wobei die Vorrichtung (1) eine Steuerungseinrichtung (8) umfasst, die die Herstellung einer Potentialdifferenz zwischen den Elektroden (4, 5, 6) steuert oder regelt, wobei mindestens drei Elektroden (4, 5, 6) am oder im Elektrodenträger (7) angeordnet sind, wobei sich die mindestens drei Elektroden (4, 5, 6) in einer Ebene (E) befinden,
**dadurch gekennzeichnet,**
**dass** mindestens eine der mindestens drei Elektroden (4, 5, 6) in ihrer Position am Elektrodenträger (7) einstellbar angeordnet ist, wobei die mindestens eine in ihrer Position einstellbare Elektrode (4, 5, 6) in der Ebene (E) translatorisch verschieblich angeordnet ist,
wobei der Elektrodenträger (7) relativ zum Halteelement (3) beweglich angeordnet ist, so dass der Elektrodenträger (7) bei am Ohr (2) angebrachtem Halteelement (3) relativ zum Halteelement (3) so verschwenken kann, dass sich alle Elektroden (4, 5, 6) im Kontakt mit der Hautoberfläche (9) der Cymba conchae befinden,
wobei zwischen dem Elektrodenträger (7) und dem Halteelement (3) ein Kugelgelenk (10) oder ein Scharniergelenk angeordnet ist oder wobei zwischen dem Elektrodenträger (7) und dem Halteelement (3) ein Federelement angeordnet ist.

2. Vorrichtung nach Anspruch 1, **dadurch gekennzeichnet, dass** die Ebene (E) zumindest näherungsweise der Hautoberfläche (9) entspricht, auf die ein transkutaner Stimulationsreiz ausgeübt werden soll.

3. Vorrichtung nach Anspruch 1 oder 2, **dadurch gekennzeichnet, dass** drei Elektroden (4, 5, 6) an oder in dem Elektrodenträger (7) dreieckförmig angeordnet sind, insbesondere in Form eines gleichseitigen Dreiecks.

4. Vorrichtung nach einem der Ansprüche 1 bis 3, **dadurch gekennzeichnet, dass** alle Elektroden (4, 5, 6) in ihrer Position am Elektrodenträger (7) einstellbar angeordnet sind.

5. Vorrichtung nach Anspruch 4, **dadurch gekennzeichnet, dass** die mindestens eine in ihrer Position einstellbare Elektrode (4, 5, 6) in einer Linearführung (11, 12, 13) linearverschieblich angeordnet ist.

6. Vorrichtung nach Anspruch 5, **dadurch gekennzeichnet, dass** bei Verstellbarkeit aller Elektroden (4, 5, 6) die Linearführungen (11, 12, 13) zueinander sternförmig ausgerichtet sind.

7. Vorrichtung nach einem der Ansprüche 1 bis 6, **dadurch gekennzeichnet, dass** das Federelement ein Verbindungsabschnitt aus dauerelastischem Material ist.

8. Vorrichtung nach einem der Ansprüche 1 bis 7, **dadurch gekennzeichnet, dass** die Verschwenkbarkeit des Elektrodenträgers (7) relativ zum Halteelement (3) um eine Längsachse (L) des Halteelements (3) unterbunden ist.

## Claims

1. Device (1) for applying a transcutaneous electrical stimulation stimulus to the surface of a section of the human ear (2), which comprises a retaining element (3) which is mountable on or in the ear (2) and a number of electrodes (4, 5, 6) which are arranged on or in an electrode carrier (7), wherein the device (1) comprises a control apparatus (8) which controls or regulates the generation of a potential difference between the electrodes (4, 5, 6), wherein at least three electrodes (4, 5, 6) are arranged on or in the electrode carrier (7), wherein the at least three electrodes (4, 5, 6) are located in one plane (E),
**characterized in**
**that** the position of at least one of the at least three electrodes (4, 5, 6) is adjustable on the electrode carrier (7), wherein the at least one electrode (4, 5, 6) of which the position is adjustable is mounted such as to be displaceable in a translational manner in the plane (E),
wherein the electrode carrier (7) is arranged movable relatively to the retaining element (3) so that the electrode carrier (7) can swivel relatively to the retaining element (3) in such a manner that all electrodes (4, 5, 6) are in contact with the skin surface (9) in the Cymba conchae when the retaining element (3) is attached to the ear (2),
wherein between the electrode carrier (7) and the retaining element (3) a spherical joint (10) or a hinge joint is arranged or wherein between the electrode carrier (7) and the retaining element (3) a spring element is arranged.

2. Device according to claim 1, **characterized in that** the plane (E) corresponds at least approximately to the skin surface (9) on which a transcutaneous stimulation stimulus has to be applied.

3. Device according to claim 1 or 2, **characterized in that** three electrodes (4, 5, 6) are arranged triangular on or in an electrode carrier (7), especially in the form of an equilateral triangle.

4. Device according to one of claims 1 to 3, **characterized in that** all electrodes (4, 5, 6) are adjustable arranged with respect to their position at the electrode carrier (7).

5. Device according to claim 4, **characterized in that** the at least one electrode (4, 5, 6) which is adjustable in its position is arranged linear movable in a linear guide (11, 12, 13).

6. Device according to claim 5, **characterized in that** in adjustablility of all electrodes (4, 5, 6) the linear guides (11, 12, 13) are aligned radiating to another.

7. Device according to one of claims 1 to 6, **characterized in that** the spring element is a connection section made from long-term flexible material.

8. Device according to one of claims 1 to 7, **characterized in that** the ability to swivel of the electrode carrier (7) relatively to the retaining element (3) is prevented around a longitudinal axis (L) of the retaining element (3).

## Revendications

1. Dispositif (1) destiné à appliquer une impulsion électrique de stimulation transcutanée sur la surface d'une partie de l'oreille humaine (2), le dispositif présentant un élément de maintien (3) qui peut être placé dans l'oreille (2) ainsi que plusieurs électrodes (4, 5, 6) disposées sur ou dans un porte-électrodes (7), le dispositif (1) comportant un ensemble de commande (8) qui commande ou régule l'établissement d'une différence de potentiel entre les électrodes (4, 5, 6), au moins trois électrodes (4, 5, 6) étant disposées sur ou dans le porte-électrodes (7), les électrodes (4, 5, 6) au nombre de trois ou davantage étant situées dans un plan (E),
**caractérisé en ce que**
la position d'au moins l'une des électrodes (4, 5, 6) au nombre de trois ou davantage sur le porte-électrodes (7) peut être ajustée, la ou les électrodes (4, 5, 6) dont la position peut être ajustée étant disposées de manière à pouvoir être déplacées en translation dans le plan (E),
**en ce que** le porte-électrodes (7) est disposé de manière à pouvoir être déplacé par rapport à l'élément de maintien (3) en inclinant le porte-électrodes (7) par rapport à l'élément de maintien (3) lorsque l'élément de maintien (3) est placé sur l'oreille (2) de telle sorte que toutes les électrodes (4, 5, 6) soient en contact avec la surface principale (9) de la Cymba conchae,
**en ce qu'**une articulation sphérique (10) ou une articulation à charnière sont disposées entre le porte-électrodes (7) et l'élément de maintien (3) et
**en ce qu'**un élément élastique est disposé entre le porte-électrodes (7) et l'élément de maintien (3).

2. Dispositif selon la revendication 1, **caractérisé en ce que** le plan (E) correspond au moins approximativement à la surface (9) de la peau sur laquelle une impulsion de stimulation transcutanée doit être appliquée.

3. Dispositif selon les revendications 1 ou 2, **caractérisé en ce que** trois électrodes (4, 5, 6) sont disposées en triangle sur ou dans le porte-électrodes (7) et en particulier sous la forme d'un triangle équilatéral.

4. Dispositif selon l'une des revendications 1 à 3, **caractérisé en ce que** toutes les électrodes (4, 5, 6) sont disposées de telle sorte que leur position sur le porte-électrodes (7) puisse être ajustée.

5. Dispositif selon la revendication 4, **caractérisé en ce que** la ou les électrodes (4, 5, 6) dont la position peut être ajustée sont disposées à déplacement linéaire dans un guide linéaire (11, 12, 13).

6. Dispositif selon la revendication 5, **caractérisé en ce que** lorsque toutes les électrodes (4, 5, 6) peuvent être ajustées, les guides linéaires (11, 12, 13) sont orientés les uns par rapport aux autres en étoile.

7. Dispositif selon l'une des revendications 1 à 6, **caractérisé en ce que** l'élément élastique est une partie de liaison en matériau à élasticité permanente.

8. Dispositif selon l'une des revendications 1 à 7, **caractérisé en ce que** la possibilité d'inclinaison du porte-électrodes (7) par rapport à l'élément de maintien (3) est limitée autour d'un axe longitudinal (L) de l'élément de maintien (3).
